# EUROPEAN PATENT APPLICATION

(11) **EP 4 131 152 A1**
(43) Date of publication of application: **08.02.2023**
(21) Application number: 20929086.5
(22) Date of filing: 31.03.2020
(51) Int. Cl.: G06T 7/00

(54) **INFORMATION PROCESSING DEVICE, INFORMATION PROCESSING METHOD, AND RECORDING MEDIUM**

(71) Applicant: NEC Corporation, 108-8001 Tokyo (JP)
(72) Inventor: HAGIWARA, Hisashi, Tokyo 108-8001 (JP); IIMURA, Yasuo, Tokyo 108-8001 (JP)
(74) Representative: Betten & Resch
(86) International application number: PCT/JP2020/014852
(87) International publication number: WO 2021/199295

(57) **Abstract**

In an information processing apparatus, genetic information unit detects genetic information from a biological sample of a person of interest. A phenotype extraction unit extracts a phenotype from an expression portion where a phenotype representing a genetic trait appears based on the genetic information. A phenotype determination unit extracts feature points from a biological image and determines a phenotype by analyzing a shape along the feature points. A matching unit matches the phenotype extracted from the biological sample with the phenotype determined from the biological image, and calculates a degree of matching between the biological sample and the biological image.

## Description

### TECHNICAL FIELD

Some non-limiting embodiments relate to a method of reducing persons of interest using genetic information.

### BACKGROUND ART

It is known that some of traits making up an appearance of a person are genetically susceptible. For instance, a so-called 'Fuji forehead' is said to be a genetic phenotype for a shape of a hairline on a head that is inherited from a parent to a child. More than 20 types of genes related to a genetic phenotype have been identified, and in recent years, a technique for creating a facial montage based on biological sample information obtained from a biological sample such as a hair or blood have been developed (Non-Patent Document 1). Such technique is believed to be useful, for instance, in creating a montage of a suspect from a blood stain left on an incident location.

### PRECEDING TECHNICAL REFERENCES

### NON-PATENT DOCUMENT

Non-Patent Document 1: PLOS GENETICS, "Modeling 3D Facial Shape from DNA", 20th March, 2014, [online], [searched on 4th October, Heisei 28], Internet <URL:http://dx.doi.org/10.1371/journal.pgen.1004224>

### SUMMARY

### PROBLEM TO BE SOLVED

However, in a case of searching for a person of interest from a biological sample, it is technically difficult to create a face montage. Also, accuracy of a created montage remains questionable for body parts where genetic effects are unlikely to appear.

It is one object of the present disclosure to provide an information processing apparatus capable of reducing persons of interest using a biological sample.

### MEANS FOR SOLVING THE PROBLEM

According to an example aspect of the present disclosure, there is provided an information processing apparatus including:
a genetic information detection unit configured to detect genetic information from a biological sample of a person of interest;
a phenotype extraction unit configured to extract a phenotype from an expression portion where a phenotype representing a genetic trait appears based on the genetic information;
a phenotype determination unit configured to extract feature points from a biological image and to determine a phenotype by analyzing a shape along the feature points;
a matching unit configured to match the phenotype extracted from the biological sample with the phenotype determined from the biological image and to calculate a degree of matching between the biological sample and the biological image.

According to another example aspect of the present disclosure, there is provided an information processing method, including:
detecting genetic information from a biological sample of a person of interest;
extracting a phenotype from an expression portion where a phenotype representing a genetic trait appears based on the genetic information;
extracting feature points from a biological image and determining a phenotype by analyzing a shape along the feature points;
matching the phenotype extracted from the biological sample with the phenotype determined from the biological image and calculating a degree of matching between the biological sample and the biological image.

According to still another example aspect of the present disclosure, there is provided a recording medium storing a program, the program causing a computer to perform a process including:
detecting genetic information from a biological sample of a person of interest;
extracting a phenotype from an expression portion where a phenotype representing a genetic trait appears based on the genetic information;
extracting feature points from a biological image and determining a phenotype by analyzing a shape along the feature points;
matching the phenotype extracted from the biological sample with the phenotype determined from the biological image and calculating a degree of matching between the biological sample and the biological image.

### EFFECT

According to the present disclosure, it becomes possible to reduce a number of persons of interest using a biological sample.

### BRIEF DESCRIPTION OF THE DRAWINGS

FIG. 1 illustrates an outline of an information processing apparatus according to a first example embodiment of the present disclosure.
FIG. 2 is a block diagram illustrating a hardware configuration of the information processing apparatus.
FIG. 3 is a block diagram illustrating a functional configuration of the information processing apparatus.
FIG. 4 illustrates an example of phenotype data of biological samples.
FIG. 5 illustrates an example of the phenotype data stored in a facial image DB.
FIG. 6 illustrates an example of a weight table indicating weights set for respective expression portions.
FIG. 7 is a flowchart of a candidate extraction process performed by the information processing apparatus.
FIG. 8 is a block diagram illustrating a functional configuration of an information processing apparatus according to a second example embodiment.

### EXAMPLE EMBODIMENTS

In the following, example embodiments of the present disclosure will be described with reference to the accompanying drawings.

### <First Example Embodiment>

### [Information Processing Apparatus]

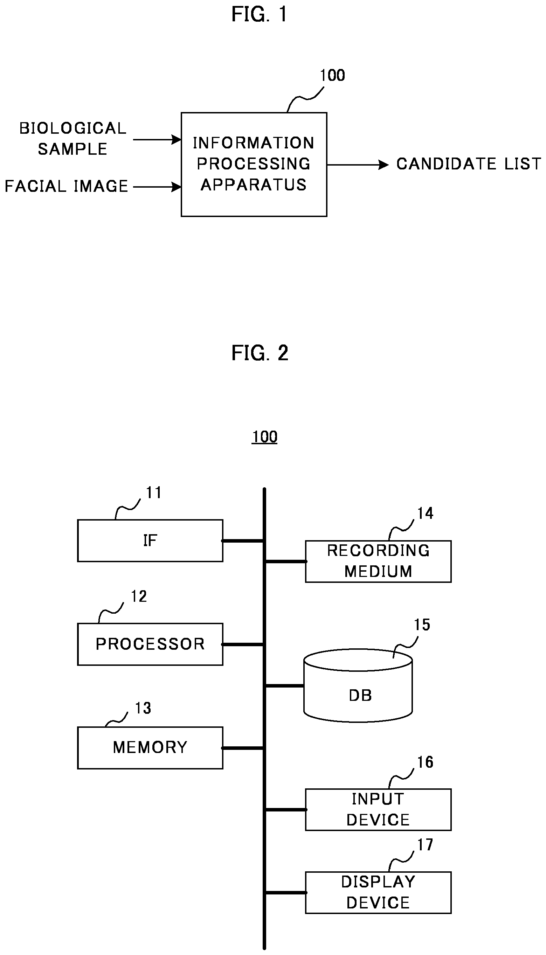

FIG. 1 illustrates an outline of an information processing apparatus according to a first example embodiment of the present disclosure. An information processing apparatus 100 reduces persons of interest based on a biological sample, and generates a candidate list of the persons of interest. Data of the biological sample of the persons of interest and facial images are input into the information processing apparatus 100. The biological sample is, for instance, blood, a hair, a part of a tissue of a body, or the like, which was left at an incident location or the like. On the other hand, the facial images may be ones registered in a database of a specific application such as a criminal database, or ones captured by security cameras or surveillance cameras installed in cities. The candidate list is a list of a plurality of candidates that are inferred to fall into a person of interest of the biological sample.

### [Hardware Configuration]

FIG. 2 is a block diagram illustrating a hardware configuration of the information processing apparatus 100. As illustrated, the information processing apparatus 100 includes an IF (InterFace) 11, a processor 12, a memory 13, a recording medium 14, a database (DB) 15, an input device 16, and a display device 17.

The IF 11 performs input and output of data. Specifically, the IF 11 acquires data of the biological sample and the facial images of a large number of people. Moreover, the IF 11 outputs a list of candidates generated by the information processing apparatus 100 to an external device as needed.

The processor 12 is a computer such as a CPU (Central Processing Unit), a GPU (Graphics Processing Unit), or the like, and controls the entire information processing apparatus 100 by executing a program prepared in advance. In particular, the processor 12 performs a candidate extraction process to be described later.

The memory 13 is formed by a ROM (Read Only Memory), RAM (Random Access Memory), or the like. The memory 13 stores various programs to be executed by the processor 12. The memory 13 is also used as a work memory during executions of various processes by the processor 12.

The recording medium 14 is a non-volatile and non-transitory recording medium such as a disk-shaped recording medium or a semiconductor memory, and is formed to be detachable from the information processing apparatus 100. The recording medium 14 records various programs which the processor 12 executes. The DB 15 stores a facial image entered from the IF 11 and phenotypes at feature points extracted from the facial image.

The input device 16 is, for instance, a keyboard, a mouse, a touch panel, or the like, and is used when a user conducts necessary instructions and inputs in connection with a process performed by the information processing device 100. The display device 17 is, for instance, a liquid crystal display, and displays a candidate list in accordance with an instruction of the user.

### [Function Configuration]

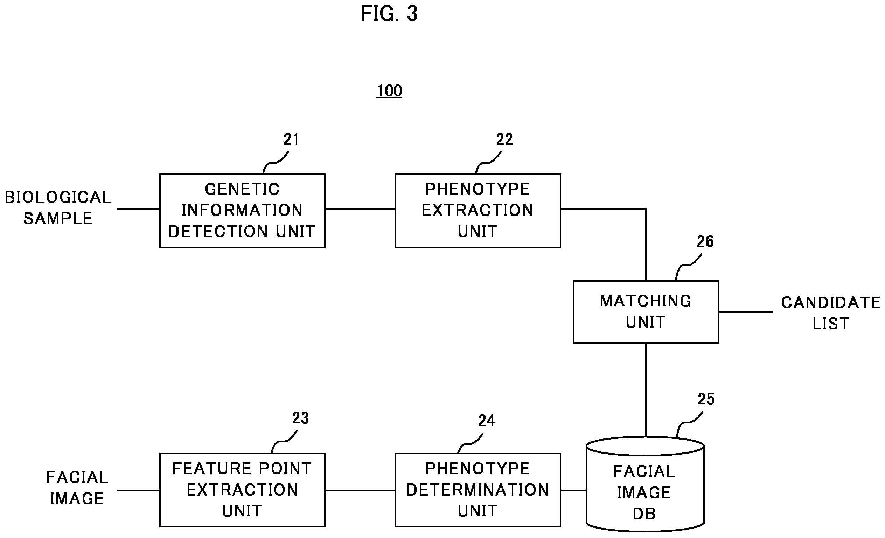

FIG. 3 is a block diagram illustrating a functional configuration of the information processing apparatus 100. The information processing apparatus 100 includes a genetic information detection unit 21, a phenotype extraction unit 22, a feature point extraction unit 23, a phenotype determination unit 24, a facial image DB 25, and a matching unit 26.

The genetic information detection unit 21 detects genetic information from data of a biological sample. The genetic information contains a so-called gene sequence. The genetic information detection unit 21 stores the detected genetic information in association with identification information of the sample.

The phenotype extraction unit 22 extracts data of genetic phenotypes based on the genetic information. A genetic phenotype (hereinafter, simply referred to as a "phenotype") refers to a trait in which a genetic trait of a person is expressed in his/her own body. Moreover, a part where the phenotype appears is called the "expression portion". The following items are known phenotypes that are easy to appear on a human appearance:
a head/face shape, a hair color, a hair thickness, hair color brightness, a hair growth, a shape or ease of hair loss, eye color, a corneal curvature, an eye function (myopia, hyperopia, astigmatism, or the like), a pupil pattern (iris), a wet/dry earwax, and the like.

For instance, it is known that whether or not a hairline on a head of a person becomes a Fuji forehead is affected by genetics. In this case, a "hairline on head" corresponds to an expression portion and a "Fuji forehead" and a "non-Fuji forehead" correspond to the phenotypes. Moreover, a phenotype that appears by dominant inheritance is called a "dominant phenotype", and a phenotype that appears by recessive inheritance is called a "recessive phenotype". Regarding the expression portion "hairline on head", the "Fuji forehead" is the dominant phenotype, and the "non-Fuji forehead" is the recessive phenotype. Note that "dominance" is also referred to as "kensei," while "recessive" is also referred to as "sensei."

Genes of a child are formed of genetics inherited from a father and a mother. At this time, a trait appearing in a phenotype is determined by a combination of genes inherited from the father and the mother. Hereafter, genes inherited from the father are called "paternal genes", and genes inherited from the mother are called "maternal genes". Moreover, a combination of genes inherited from the father and the mother is called a "genotype".

It is known that an arrangement at which position in the gene sequence genetically influences on which expression portion. Hence, the phenotype extraction unit 22 refers to a predetermined position of the gene sequence, and extracts an arrangement of that position as the phenotype of an expression portion corresponding to that position. By referring to the predetermined position, a process load is reduced. The phenotype extraction unit 22 expresses the extracted gene sequence as the phenotype by text information.

Specifically, it is assumed that an eight-base part at a certain position of the gene sequence indicates a genotype matching to the phenotype in a gene sequence that determines a hairline on a head. A phenotype of an expression portion A "hairline on head" corresponds to either the dominant phenotype "Fuji forehead" or the recessive phenotype "non-Fuji forehead". Here, it is assumed that an arrangement of the dominant genotype is "TTGTTTCG" and that an arrangement of the recessive genotype is "CCAGGGAC." In this case, the following three patterns correspond to the phenotype expressing the dominant "Fuji forehead."
(1) Paternal gene sequence: "TTGTTTCG"
   Maternal gene sequence: "TTGTTTCG"
(2) Paternal gene sequence: "TTGTTTCG"
   Maternal gene sequence: "CCAGGGAC"
(3) Paternal gene sequence: "CCAGGGAC"
   Maternal gene sequence: "TTGTTTCG"
   Also, the following one pattern corresponds to the phenotype expressing the recessive "non-Fuji forehead".
(4) Paternal gene sequence: "CCAGGGAC"
   Maternal gene sequence: "CCAGGGAC"

In the following, the genotype in an expression portion is indicated by an uppercase and a lowercase of alphabet letters. For instance, for the expression portion A, the dominant inheritance is indicated by "A" and the recessive inheritance is indicated by "a". Also, the genotype is expressed in an order of paternal genes and maternal genes. For instance, if the paternal gene is "A" and the mother gene is "a", the genotype is referred to as "Aa". In this case, for the phenotype A indicating a hairline on a head, the gene A = "TTGTTTCG" and the gene a = "CCAGGGAC". When the genotype is "AA," "Aa," or "aA," the phenotype is the "Fuji forehead," and when the phenotype is "aa," the phenotype is the "non-Fuji forehead."

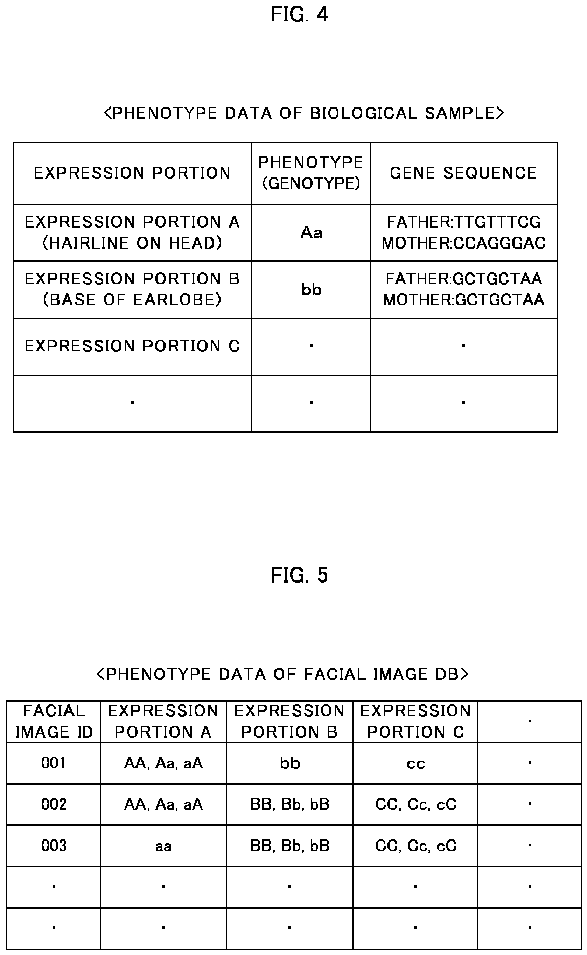

FIG. 4 illustrates an example of phenotype data of biological samples obtained by the phenotype extraction unit 22. In these biological samples, a paternal gene sequence obtained from a predetermined position of genetic information is "TTGTTTCG", and a maternal gene sequence is "CCAGGGAC". Hence, the phenotype (genotype) of the expression portion A is indicated by "Aa". Similarly, for each of other phenotype positions, the phenotype is determined based on the paternal gene sequence and the maternal gene sequence obtained from a predetermined position of the genetic information. Thus, the phenotype extraction unit 22 extracts the phenotype for each phenotype portion based on the genetic information.

Returning to FIG. 3, the feature point extraction unit 23 performs an image analysis with respect to an input facial image and extracts feature points corresponding to each expression portion to be extracted from a biological sample. Specifically, the feature point extraction unit 23 extracts feature points corresponding to each of expression portions such as the expression portion A (hairline on head) and an expression portion B (base of earlobe) from the facial image. The feature point extraction unit 23 extracts respective feature points by, for instance, a pattern matching. The feature point extraction unit 23 outputs an image of the extracted feature points to the phenotype determination unit 24.

The phenotype determination unit 24 performs the image analysis of a shape along the feature points based on the image from which the feature points are extracted by the feature point extraction unit 23, and determines the phenotype represented by these feature points. For instance, the phenotype determination unit 24 determines whether a hairline in a facial image represents the "Fuji forehead" or the "non-Fuji forehead", based on the image of the feature points corresponding to the expression portion A (hairline on head). When the facial image represents the "Fuji forehead", the phenotype determination unit 24 determines that the phenotype of the expression portion A is one of the above described "AA," "Aa," or "aA." On the other hand, when the facial image represents the "non-Fuji forehead", the phenotype determination unit 24 determines that the phenotype of the expression portion A is "aa". Accordingly, the phenotype determination unit 24 determines the phenotype of the expression portion corresponding to the feature points extracted from the facial image. Incidentally, the phenotype determination unit 24 may determine the phenotype by the image analysis using the pattern matching or the like, or may determine the phenotype by using a model learned in advance using machine learning or the like. The phenotype determination unit 24 records the phenotype for each expression portion in the facial image DB 25 in association with the identification information such as an ID of the facial image.

FIG. 5 illustrates an example of the phenotype data stored in the facial image DB 25. For instance, since the dominant phenotype is any of the three patterns ("AA", "Aa", and "aA") for the expression portion A (hairline on head), the three patterns are stored for the facial image in which the hairline is the "Fuji forehead". On the other hand, since the recessive phenotype is limited to a single pattern "aa", the single pattern is stored for the facial image in which the hairline is the "non-Fuji forehead". Similarly, for other expressions, three patterns are stored for the dominant phenotype and one pattern is stored for the recessive phenotype.

The matching unit 26 matches the phenotype of the biological sample extracted by the phenotype extraction unit 22 with the phenotype of the facial image that is determined by the phenotype determination unit 24 and stored in the facial image DB 25, and calculates a degree of matching. Specifically, the matching unit 26 compares the phenotype of the biological sample with the phenotype of the facial image for each expression portion, and determines whether or not the phenotype of the biological sample matches the phenotype of the facial image. Incidentally, in a case where the phenotype of the facial image includes three patterns for the dominant phenotype, when the phenotype of the biological sample is included in the three patterns, the matching unit 26 determines that the phenotype of the biological sample matches the phenotype of the facial image.

For instance, a case is considered in which the phenotype of the biological sample shown in FIG. 4 is compared with the phenotype of the facial image of a facial image ID "001" shown in FIG. 5. Since the phenotype "Aa" of the expression portion A of the biological sample is included in the phenotypes "AA, Aa, aA" for the expression portion A in the facial image of the facial image ID "001", the matching unit 26 determines that the phenotype of the biological sample and the phenotype of the facial image are matched with each other for the expression portion A. Similarly, with respect to the expression portion B, since a phenotype "bb" of the biological sample matches with the phenotype "bb" of the facial image of the facial image ID "001", the matching unit 26 determines that the phenotype of the biological sample and the phenotype of the facial image are matched with each other for the expression portion B. By this manner, the matching unit 26 conducts matching of the phenotype of the biological sample with the phenotype of the facial image for each expression portion.

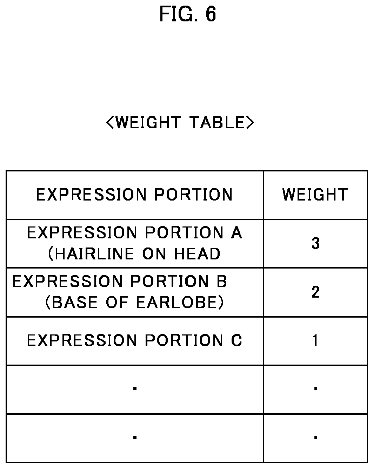

For instance, in a case where the phenotype of the biological sample matches the phenotype of the facial image for one expression portion, the matching unit 26 sets "1" to a degree of matching for the expression portion, and sets "0" to the degree of matching for the expression portion in a case where these phenotypes are mismatched. Then, the matching unit 26 aggregates degrees of matching according to respective expression portions (hereinafter, referred to as "degrees of matching for respective expression portions"). Here, the matching unit 26 calculates a total of the degrees of matching for respective expression portions using a predetermined weight for each expression portion. FIG. 6 illustrates an example of a weight table indicating weights set for respective expression portions. A value of a weight for each expression portion is set according to the susceptibility of the genetic influence of that expression portion. Specifically, a greater weight is set at the expression portion that is considered to be susceptible to genetic influences. In the example of FIG. 6, it is shown that the expression portions A, B, and C are susceptible to genetic influence in this order. The weights shown in FIG. 6 are given for convenience of explanation, and do not indicate that a hair growth is actually more susceptible to genetic influence than the base of the earlobe.

The matching unit 26 calculates an overall degree of matching (hereinafter, also referred to as a "total degree of matching") between the phenotype of a certain biological sample and the phenotype of one facial image by multiplying degrees of matching for expression portions each being "1" or "0" at expression portions by weights corresponding to the expression portions and aggregating the multiplied degrees. Since the susceptibility to genetic influence is taken into consideration by using weights, it is possible to improve reduction accuracy of candidates of the facial images. Then, when the calculated total degree of matching is greater than a predetermined threshold value, the facial image is determined as a candidate corresponding to the biological sample. In this process, the matching unit 26 compares the phenotype of a certain biological sample with the phenotype of a plurality of facial images stored in the facial image DB 25, and outputs a list of facial images determined as candidates corresponding to the biological sample as a candidate list.

According to the information processing apparatus of the present example embodiment, by comparing the phenotype acquired from the genetic information of the biological sample with the phenotype determined by the image analysis concerning the facial image, it is possible to refine candidates of the facial image corresponding to the biological sample. Therefore, it becomes possible to reduce a number of candidates corresponding to the biological sample from a large number of facial images.

### [Candidate Selection Process]

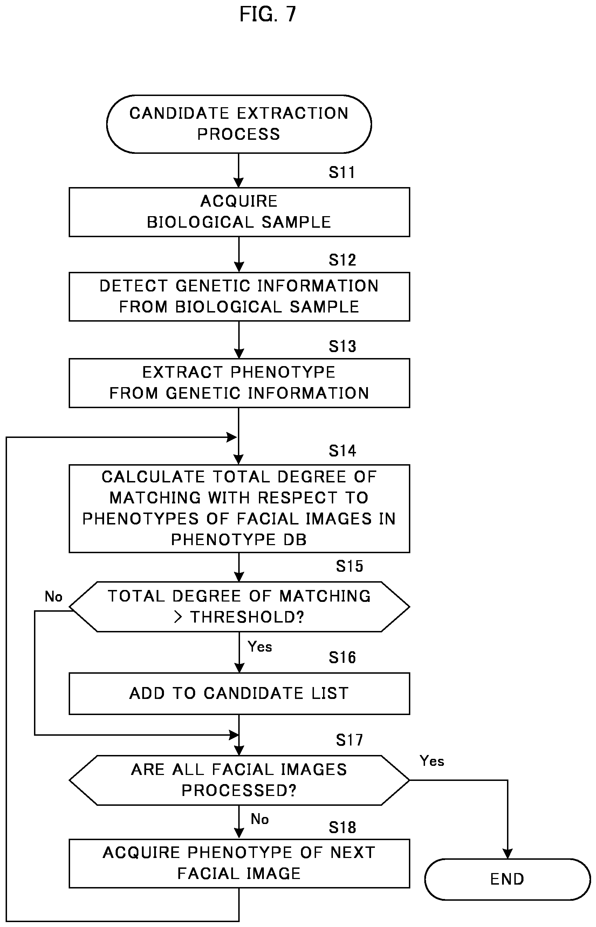

FIG. 7 is a flowchart of a candidate extraction process performed by the information processing apparatus 100. This process is realized by the processor 12 shown in FIG. 2 executing a program prepared in advance and operating as each element shown in FIG. 3. As a premise of this process, a phenotype is determined with respect to an expression portion included in a plurality of facial images, and the phenotype data as illustrated in FIG. 5 is stored in the facial image DB 25.

First, the genetic information detection unit 21 acquires a biological sample (step S11), and detects the genetic information including a gene sequence from the biological sample (step S12). Next, the phenotype extraction unit 22 refers to a predetermined position of the gene sequence, and extracts the phenotype for each expression portion (step S13). Accordingly, the phenotype data of the biological sample are generated as illustrated in FIG. 4.

Next, the matching unit 26 compares, at each of expression portions, the phenotype of the biological sample with the phenotypes of the plurality of facial images stored in the facial image DB 25, thus calculates degrees of matching for respective expression portions, and refers to the weight table illustrated in FIG. 6, and further calculates a total degree of matching by weighting and aggregating the degrees of matching for respective expression portions (step S14). Subsequently, the matching unit 26 determines whether the total degree of matching is greater than a predetermined threshold value (step S15). When the total degree of matching is not greater than a predetermined threshold value (step S15: No), this process advances to step S17. On the other hand, when the total degree of matching is greater than the predetermined threshold value (step S15: Yes), the matching unit 26 adds the facial image into the candidate list (step S16) and advances to step S17.

In step S17, the matching unit 26 determines whether or not all the facial images of interest have been processed. When all the facial images are not processed (step S17: No), the matching unit 26 acquires the phenotype of a next facial image from the facial image DB 25 and repeats a process from steps S14 to S17. On the other hand, when all the facial images are processed (step S17: Yes), this matching process is terminated.

Accordingly, for all the facial images of interest, the total degree of matching with respect to the phenotype of the biological sample is calculated, and a facial image, which total degree of matching is greater than the predetermined threshold value, is added into the candidate list. Hence, at an end of the candidate extraction process, a facial image having a high degree of matching with the phenotype obtained based on the biological sample is extracted as a candidate. Therefore, it is possible to reduce a number of candidates as targets corresponding to a biological sample from a large number of the facial images.

### [Modification Example]

### (Modification Example 1)

In the example embodiment described above, matching of the phenotype is conducted for each expression portion in a predetermined order, for instance, in an order of the expression portions A -> B -> C. On the other hand, in the modification examples, focusing on a point that recessive genotypes are less than dominant genotypes, by preferentially performing matching at an expression portion representing the recessive phenotype, the number of candidates corresponding to the biological sample are reduced more efficiently.

In a case of matching by combining phenotypes at a plurality of expression portions, a ratio at which all phenotypes are recessive is smaller than a ratio at which not all of them are recessive. For instance, it is assumed that two expression portions are used: the expression portion A "hairline on head" and the expression portion B "base of earlobe". According to Mendel's laws of dominance, a separation ratio between the dominant genotype and the recessive genotype can be indicated by a frequency of a genotype included in each phenotype.

In the above example, when cases are assumed as follows:
(Case 1) Both dominant (Fuji forehead, earlobe hanging),
(Case 2) The expression portion A is dominant, and the expression portion B is recessive (Fuji forehead, earlobe not hanging),
(Case 3) The expression portion A is recessive, and expression portion B is dominant (non-Fuji forehead, earlobe hanging) and
(Case 4) Both recessive (non-Fuji forehead, earlobe not hanging), a ratio of Case 1: Case 2: Case 3: Case 4 is 9:3:3: 1. That is, in a case of using two expression portions, a probability that both are recessive is 1/16 of the total. Incidentally, in a case of using three expression portions, a probability that all are recessive is 1/64 of the total. As described above, a rate that an expression portion shows the recessive phenotype, especially a rate that all expression portions show the recessive phenotype, becomes considerably smaller than a rate that not all of them show the recessive phenotype.

Accordingly, in the modification example 1, in a case where the recessive phenotype is included in phenotypes of expression portions extracted from a biological sample, the matching process by the matching unit 26 is performed by giving priority to an expression portion indicating the recessive phenotype. Specifically, in a case where there is an expression portion indicating the recessive phenotype in phenotypes of a certain biological sample, the matching unit 26 performs the matching for that expression portion in priority to expression portions not indicating the recessive phenotype. For instance, in a case where the matching unit 26 performs the matching using the three expression portions A to C, when the phenotype of the expression portion A obtained from the biological sample is not recessive (one of AA, Aa, and aA), the phenotype of the expression portion B is recessive (bb), and the phenotype of the expression portion C is recessive (cc), the matching unit 26 performs the matching for the expression portions B and C prior to the expression portion A. That is, the matching unit 26 performs the matching of the phenotype for each expression position in an order of the expression portions B -> C -> A or C -> B -> A, rather than an order of the expression portions A -> B -> C. In this order, since a number of facial images to be matched is greatly reduced at an early stage by the matching with the recessive phenotype that is preferentially performed, it is possible to perform the matching between the phenotype of the biological sample and the phenotypes of the facial images more efficiently.

### (Modification Example 2)

The display device 17 can display a part of or all of information output from each configuration of the information processing apparatus 100. Also, the display device 17 may display an original image in which the feature point extraction unit 23 has extracted feature points, and may highlight, at that time, the feature points where the matching has been successfully conducted on a facial image. Moreover, as a result of reducing the facial images by the information processing apparatus 100, the display device 17 may list sets of person information concerning the reduced candidates as a candidate list. At that time, the display device 17 may display each candidate in an order of higher degrees of matching. At that time, the display device 17 may display each candidate together with at least one of the person information, the degree of matching, and information (such as a specific shape of a forehead or an ear) indicating an expression portion of the recessive where the matching has been successfully conducted.

### (Modification Example 3)

In the above example embodiment, a portion of a face is used as the expression portion where the phenotype appears; however, a portion where the phenotype showing a genetic trait of a biological body appears is not limited to the face. For instance, since there are expression portions where a phenotype appears other than a face, such as a shape of a nail (a vertical nail or a horizontal nail) and a curl of a thumb, it is also possible to use the phenotype of such the expression portions in the information processing apparatus of the present disclosure. That is, besides the face, it is possible to perform the matching of the phenotype using a biological image showing a shape, a property, and a feature of a hand, a foot, an arm, a body, a body surface, a skin, a nail, a finger, or the like.

### <Second Example Embodiment>

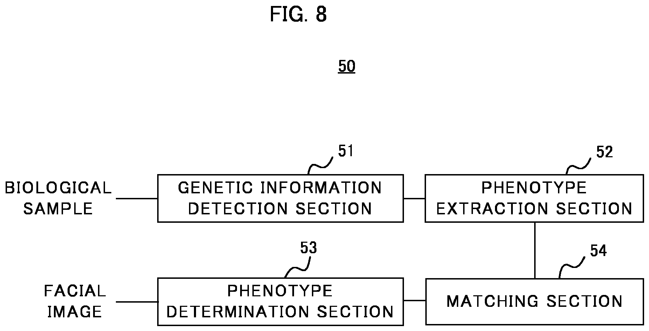

FIG. 8 is a block diagram illustrating a functional configuration of an information processing apparatus 50 according to a second example embodiment. The information processing apparatus 50 includes a genetic information detection section 51, a phenotype extraction section 52, a phenotype determining section 53, and a matching section 54. The genetic information detection section 51 detects genetic information from a biological sample of a person of interest. The phenotype extraction section 52 extracts a phenotype from an expression portion where the phenotype showing a genetic trait appears based on the genetic information. The phenotype determination section 53 extracts feature points where the phenotype appears from a facial image, and analyzes a shape of the feature points to determine the phenotype. The matching section 54 compares the phenotype extracted from the biological sample with the phenotype determined from the facial image, and calculates a degree of matching between the biological sample and the facial image.

A part or all of the example embodiments described above may also be described as the following supplementary notes, but not limited thereto.

### (Supplementary note 1)

An information processing apparatus comprising:
a genetic information detection unit configured to detect genetic information from a biological sample of a person of interest;
a phenotype extraction unit configured to extract a phenotype from an expression portion where a phenotype representing a genetic trait appears based on the genetic information;
a phenotype determination unit configured to extract feature points from a biological image and to determine a phenotype by analyzing a shape along the feature points;
a matching unit configured to match the phenotype extracted from the biological sample with the phenotype determined from the biological image and to calculate a degree of matching between the biological sample and the biological image.

### (Supplementary note 2)

The information processing apparatus according to supplementary note 1, wherein the biological image is a facial image.

### (Supplementary note 3)

The information processing apparatus according to supplementary note 2, wherein
the matching unit stores weights determined beforehand for respective expression portions, and
a degree of matching is calculated for each expression portion by using a value indicating the phenotype extracted from the biological sample, a value indicating the phenotype determined from the facial image, and the weights, and the degree of matching between the biological sample and the facial image is calculated by aggregating degrees of matching for all expression portions.

### (Supplementary note 4)

The information processing apparatus according to supplementary note 2 or 3, wherein the matching unit is configured to output a facial image which degree of matching with respect to the biological sample is greater than a predetermined threshold among a plurality of facial images, as a candidate of a facial images corresponding to the biological sample.

### (Supplementary note 5)

The information processing apparatus according to any one of supplementary notes 2 through 4, wherein the phenotype extraction unit is configured to extract the phenotype by referring to a predetermined position corresponding to the expression portion in the genetic information.

### (Supplementary note 6)

The information processing apparatus according to any one of supplementary notes 2 through 5, wherein the matching unit is configured to extract a recessive phenotype from among phenotypes extracted from the biological sample and to match a phenotype of the expression portion representing the recessive phenotype with priority over phenotypes of other expression portions.

### (Supplementary note 7)

The information processing apparatus according to any one of supplementary notes 2 through 6, further comprising a database configured to store, for each of a plurality of facial images, a value of the phenotype detected from the facial image at each expression portion in association with identification information of the facial image.

### (Supplementary note 8)

An information processing method, comprising:
detecting genetic information from a biological sample of a person of interest;
extracting a phenotype from an expression portion where a phenotype representing a genetic trait appears based on the genetic information;
extracting feature points from a biological image and determining a phenotype by analyzing a shape along the feature points;
matching the phenotype extracted from the biological sample with the phenotype determined from the biological image and calculating a degree of matching between the biological sample and the biological image.

### (Supplementary note 9)

A recording medium storing a program, the program causing a computer to perform a process comprising:
detecting genetic information from a biological sample of a person of interest;
extracting a phenotype from an expression portion where a phenotype representing a genetic trait appears based on the genetic information;
extracting feature points from a biological image and determining a phenotype by analyzing a shape along the feature points;
matching the phenotype extracted from the biological sample with the phenotype determined from the biological image and calculating a degree of matching between the biological sample and the biological image.

While the disclosure has been described with reference to the example embodiments and examples, the disclosure is not limited to the above example embodiments and examples. It will be understood by those of ordinary skill in the art that various changes in form and details may be made therein without departing from the spirit and scope of the present disclosure as defined by the claims.

### DESCRIPTION OF SYMBOLS

- 11: IF (InterFace)
- 12: Processor
- 13: Memory
- 14: Recording medium
- 15: Database (DB)
- 16: Input device
- 17: Display device
- 21: Genetic information detection unit
- 22: Phenotype extraction unit
- 23: Feature point extraction unit
- 24: Phenotype determination unit
- 25: Facial image DB
- 26: Matching unit
- 50: Genetic information detection section
- 51: Genetic information detection section
- 52: Phenotype extraction section
- 53: Phenotype determination section
- 54: Matching section

## Claims

1. An information processing apparatus comprising:
a genetic information detection unit configured to detect genetic information from a biological sample of a person of interest;
a phenotype extraction unit configured to extract a phenotype from an expression portion where a phenotype representing a genetic trait appears based on the genetic information;
a phenotype determination unit configured to extract feature points from a biological image and to determine a phenotype by analyzing a shape along the feature points;
a matching unit configured to match the phenotype extracted from the biological sample with the phenotype determined from the biological image and to calculate a degree of matching between the biological sample and the biological image.

2. The information processing apparatus according to claim 1, wherein the biological image is a facial image.

3. The information processing apparatus according to claim 2, wherein
the matching unit stores weights determined beforehand for respective expression portions, and
a degree of matching is calculated for each expression portion by using a value indicating the phenotype extracted from the biological sample, a value indicating the phenotype determined from the facial image, and the weights, and the degree of matching between the biological sample and the facial image is calculated by aggregating degrees of matching for all expression portions.

4. The information processing apparatus according to claim 2 or 3, wherein the matching unit is configured to output a facial image which degree of matching with respect to the biological sample is greater than a predetermined threshold among a plurality of facial images, as a candidate of a facial images corresponding to the biological sample.

5. The information processing apparatus according to any one of claims 2 through 4, wherein the phenotype extraction unit is configured to extract the phenotype by referring to a predetermined position corresponding to the expression portion in the genetic information.

6. The information processing apparatus according to any one of claims 2 through 5, wherein the matching unit is configured to extract a recessive phenotype from among phenotypes extracted from the biological sample and to match a phenotype of the expression portion representing the recessive phenotype with priority over phenotypes of other expression portions.

7. The information processing apparatus according to any one of claims 2 through 6, further comprising a database configured to store, for each of a plurality of facial images, a value of the phenotype detected from the facial image at each expression portion in association with identification information of the facial image.

8. An information processing method, comprising:
detecting genetic information from a biological sample of a person of interest;
extracting a phenotype from an expression portion where a phenotype representing a genetic trait appears based on the genetic information;
extracting feature points from a biological image and determining a phenotype by analyzing a shape along the feature points;
matching the phenotype extracted from the biological sample with the phenotype determined from the biological image and calculating a degree of matching between the biological sample and the biological image.

9. A recording medium storing a program, the program causing a computer to perform a process comprising:
detecting genetic information from a biological sample of a person of interest;
extracting a phenotype from an expression portion where a phenotype representing a genetic trait appears based on the genetic information;
extracting feature points from a biological image and determining a phenotype by analyzing a shape along the feature points;
matching the phenotype extracted from the biological sample with the phenotype determined from the biological image and calculating a degree of matching between the biological sample and the biological image.
